Europäisches Patentamt

European Patent Office

Office européen des brevets

(11) Publication number: **0 307 860 B1**

# EUROPEAN PATENT SPECIFICATION

(45) Date of publication of patent specification: **08.06.94**   (51) Int. Cl.⁵: **C07K 7/10, A61K 37/02**

(21) Application number: **88114948.8**

(22) Date of filing: **13.09.88**

(54) **Cyclic GRF-analogs.**

(30) Priority: **18.09.87 US 98340**

(43) Date of publication of application:
**22.03.89 Bulletin 89/12**

(45) Publication of the grant of the patent:
**08.06.94 Bulletin 94/23**

(84) Designated Contracting States:
**AT BE CH DE ES FR GB IT LI LU NL SE**

(56) References cited:
**EP-A- 0 136 475**
**EP-A- 0 188 214**
**EP-A- 0 292 334**

**J. Clin. Invest., 83, 1989, 1533-1540, Frohman et al.**

(73) Proprietor: **F. HOFFMANN-LA ROCHE AG**
**Postfach 3255**
**CH-4002 Basel(CH)**

(72) Inventor: **Felix, Arthur Martin**
**257 Central Avenue**
**West Caldwell N.J. 07006(US)**
Inventor: **Heimer Edgar Philip**
**87 Edison Terrace**
**Sparta N.J. 07871(US)**

(74) Representative: **Lederer, Franz, Dr. et al**
**Lederer, Keller & Riederer**
**Patentanwälte**
**Prinzregentenstrasse 16**
**D-80538 München (DE)**

# EP 0 307 860 B1

**Description**

Growth in animals is believed to be regulated by a cascade of bio-regulatory molecules. The hypothalamus produces a substance called Growth Hormone Releasing Factor (GRF) which in turn acts upon the pituitary to cause release of growth hormone. The pituitary is maintained under negative feedback control by somatostatin and insulin growth factor (IGF). GRF has been found to be enormously active, and capable of stimulating the release of microgram per ml levels of growth hormone in the blood. GRF can be utilized therapeutically in most of the areas now considered as candidates for the application of growth hormone, for example in the treatment of pituitary dwarfism and diabetes resulting from abnormal growth hormone production, enhancement of wound healing, treatment of burns, or retardation of the aging process.

The successful isolation of GRF was due partly to the discovery that pancreatic tumors associated with acromegaly ectopically produced large quantities of GRF. Three forms of GRF with homologous amino acid sequence but different length (44, 40, and 37 amino acids) have been isolated.

The amidated form of GRF with 44 amino acids is considered to be the parent molecule. A wide variety of synthetic analogs have been produced. They consist of polypeptides having an amino acid sequence equivalent to the one of the original isolated GRF or of biologically active fragments or analogs thereof with various amino acid substitutions. The changes have been specifically engineered to often yield synthetic analogs with biological properties superior to those of the parent molecule. Accordingly, the desire has been to engineer GRF analogs which exhibit maximum biological activity in terms of potency, effectiveness, and stability, for example.

To date, all of the known GRF analogs are of linear configuration. For example, EP-A-0 292 334 discloses various linear synthetic GRF analogues which are said to be potent in stimulating the release of pituitary growth hormone in animals, including humans. Generally, linear peptides are very flexible molecules and lack a well-defined conformation. Each amino acid in a linear peptide is exposed to the surrounding milieu resulting in greater susceptibility to enzymatic and chemical degradation.

A cyclic peptide (lactam) is a peptide wherein the side-chain carboxy terminus of an acidic amino acid (e.g. Asp or Glu) is attached to the side-chain amino terminus of a basic amino acid (e.g. Lys) via the generation of an amide bond.

The biological properties of cyclic peptides are often altered relative to those of their linear analogs. Cyclic peptides are much more rigid, with well defined shapes and interior amino acid residues which are shielded from the surrounding milieu. These changes are reflected in the biological properties of the peptide. The cyclic peptides' duration of action will be longer since the compact structure renders it less susceptible to chemical and enzymatic degradation. The bioavailability of the cyclic peptide will be increased due to changes in the tissue distribution caused by the shielded interior amino acid residues. Further, the well defined conformation of the cyclic peptide will give it greater specificity for the target receptor thus reducing the probability of undesirable biological side effects. In the case of linear peptides for example there is generally cross reactivity for a given linear peptide with central and peripheral receptors and there is also considerable cross reactivity of a given peptide with receptors for another peptide.

The instant invention is directed to linear and cyclic analogs of GRF of the specific amino acid sequence set forth herein, including the pharmaceutically acceptable salts thereof.

The instant invention is also directed to a method of stimulating the release of growth hormone in a subject by administering to the subject an effective amount of the compounds of the invention.

The following symbols and terminology as utilized in this specification shall be defined as follows:

1. cyclic peptide - or lactam means a peptide wherein the side-chain carboxy terminus of an acidic amino acid (e.g. Asp or Glu) is attached to the side-chain, amino terminus of a basic amino acid (e.g. Lys) via an amide bond (lactam).

2.

```
        8                12
        A                 B
        L_____J
```

$\underline{\text{or cyclo}}\ ^{8,12}$

means that the eighth amino acid "A" in the peptide chain is attached to the twelfth amino acid "B" in the chain to yield a cyclic lactam structure.

3. GRF means human growth hormone releasing factor which is a polypeptide having the amino acid sequence

```
                                   5
    Tyr-Ala-Asp-Ala-Ile-Phe-Thr-Asn-Ser-
          10                  15
    Tyr-Arg-Lys-Val-Leu-Gly-Gln-Leu-Ser-Ala-
    20                          25
    Arg-Lys-Leu-Leu-Gln-Asp-Ile-Met-Ser-
          30                          35
    Arg-Gln-Gln-Gly-Glu-Ser-Asn-Gln-Glu-Arg-
          40
    Gly-Ala-Arg-Ala-Arg-Leu-NH2
```

4. [Leu$^{27}$]GRF means a polypeptide having an amino acid sequence corresponding to GRF in which a methionine residue at position 27 has been substituted by a leucine residue. Such analogs of GRF are generally indicated by setting forth the substituted amino acid in brackets before GRF.

5. GRF (1-29) means a fragment of the GRF peptide having the first 29 amino acids of the full sequence. In general, numbers in parenthesis following GRF correspond to the positions of amino acids of the original GRF sequence indicating N-terminus and C-terminus of the fragment.

6. des NH$_2$Tyr$^1$ means that the amino terminal NH$_2$ group is removed from the Tyrosine residue at position 1.

7. OcHex means cyclohexl

8. DIEA means diisopropylethylamine

8. DTE means dithioethane

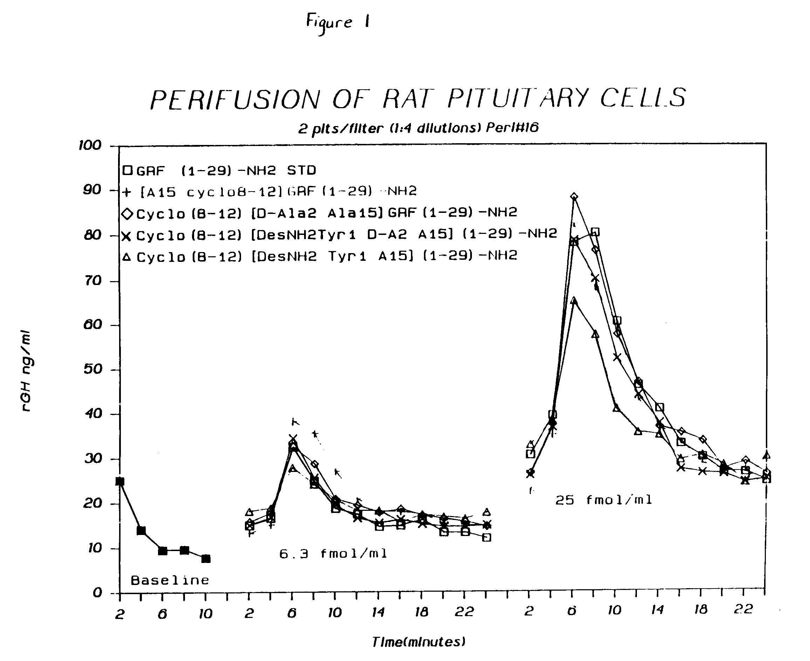

Figure 1 illustrates the effectiveness of the compounds of the invention in the release of growth hormone from rat pituitary cells. Two pituitary ("pits") glands (1:4 dilutions) were impregnated onto each filter of the filter assay which assay measures biological activity in general. The following specific abbreviations are used:

"rGH" means recombinant growth hormone, "[A15 cyclo 8-12]GRF(1-29)-NH2" means Cyclo$^{8,12}$-[Asp$^8$,Ala$^{15}$]-GRF-(1-29)-NH$_2$, "Cyclo(8-12)[D-Ala2 Ala15]GRF(1-29)-NH2" means Cyclo$^{8,12}$[D-Ala$^2$,Asp$^8$,Ala$^{15}$]-GRF-(1-29)-NH$_2$, "Cyclo (8-12)[DesNH2Tyr1 D-A2 A15](1-29)-NH2" means Cyclo$^{8,12}$-[desNH$_2$Tyr$^1$,D-Ala$^2$,Asp$^8$,Ala$^{15}$]-GRF-(1-29)-NH$_2$ and "Cyclo (8-12)[DesNH2Tyr1A15] (1-29)-NH2" means Cyclo$^{8,12}$[desNH$_2$Tyr$^1$,Asp$^8$,Ala$^{15}$]-GRF-(1-29)-NH$_2$.

The instant invention comprises cyclic peptides of the formula

```
I        R1-R2-Asp-Ala-Ile-Phe-Thr-A-Ser-Tyr-Arg-B-Val-Leu
                                      |_____|

         -R3-Gln-Leu-Ser-Ala-Arg-Lys-Leu-Leu-Gln-Asp-Ile

         -R4-R5-R6-X
```

wherein

R$^1$ = Tyr, desNH$_2$-Tyr, Ac-Tyr, His, N-Methyl-L-Tyr

R$^2$ = Ala, D-Ala, N-Methyl-D-Ala

R$^3$ = Gly, Ala, Leu, Val, Ile, Nle, NVal, β-Ala, α-Aib

R$^4$ = Met, Leu, Nle, Ile

R$^5$ = Ser, Asn

R$^6$ = an amino acid sequence selected from the group consisting of Arg-Gln-Gln-Gly-Glu-Ser-Asn-Gln-Glu-Arg-Gly-Ala-Arg-Ala-Arg-Leu or fragments thereof where the fragment is reduced in number by 1 to 15 amino acids from the carboxyl end

X = OH, $NH_2$, $N(R^7)$ $(R^8)$ where $R^7$ and

$R^8$ = H or lower alkyl;

A = Asp, Glu, $\alpha$-aminoadipic acid, $\alpha$-aminopimelic acid

B = Lys, Orn, diaminopropionic acid, diaminobutyric acid

with the side chain carboxy terminus of A is bonded via an amide bond to the side chain amino terminus of B,

and the pharmaceutically acceptable salts thereof.

Preferred are peptides of Formula I wherein $R^1$ = Tyr, $desNH_2$-Tyr, N-methyl-L-Tyr; $R^2$ = Ala, D-Ala; $R^3$ = Ala; and X = $NH_2$.

Further preferred is a peptide according to Formula I like above wherein A = Asp; B = Lys (and the side-chain carboxy terminus of Asp is covalently linked to the side-chain amino terminus of Lys via an amide bond); $R^4$ = Met; $R^5$ = Ser; and $R^6$ = Arg.

Particularly preferred is a peptide wherein $R^1$ = Tyr, $R^2$ = Ala, and $R^3$ = Ala with the formula

```
Tyr-Ala-Asp-Ala-Ile-Phe-Thr-Asp-Ser-Tyr-Arg-Lys-Val-Leu-Ala-
Gln-Leu-Ser-Ala-Arg-Lys-Leu-Leu-Gln-Asp-Ile-Met-Ser-Arg-NH₂.
```

Also particularly preferred is a peptide wherein $R^2$ = D-Ala with the formula

```
Tyr-D-Ala-Asp-Ala-Ile-Phe-Thr-Asp-Ser-Tyr-Arg-Lys-Val-Leu-Ala-
Gln-Leu-Ser-Ala-Arg-Lys-Leu-Leu-Gln-Asp-Ile-Met-Ser-Arg-NH₂.
```

Also preferred are peptides according to Formula I wherein $R^1$ = $desNH_2$Tyr.

Particularly preferred is such a peptide wherein $R^2$ = Ala said peptide having the formula

```
desNH₂-Tyr-Ala-Asp-Ala-Ile-Phe-Thr-Asp-Ser-Tyr-Arg-Lys-Val-
Leu-Ala-Gln-Leu-Ser-Ala-Arg-Lys-Leu-Leu-Gln-Asp-Ile-Met-Ser-
Arg-NH₂
```

Also particularly preferred is such a peptide wherein $R^2$ = D-Ala with the formula

```
desNH₂-Tyr-D-Ala-Asp-Ala-Ile-Phe-Thr-Asp-Ser-Tyr-Arg-Lys-
Val-Leu-Ala-Gln-Leu-Ser-Ala-Arg-Lys-Leu-Leu-Gln-Asp-Ile-Met-Ser-
Arg-NH₂
```

The peptides can be synthesized by suitable methods such as exclusive solid phase synthesis, partial solid phase methods, fragment condensation or classical solution synthesis. Recombinant DNA techniques can also be used for analogs containing only natural amino acid residues. The peptides of the invention are preferably prepared by solid phase peptide synthesis as described by Merrifield, J. Am. Chem. Soc. 85, 2149 (1963). The synthesis is carried out with amino acids protected at the alpha-amino-terminus. Trifunctional amino acids with labile side-chains are also protected with suitable groups which will prevent a chemical reaction from occurring at that site during the assemblage of the peptide. The alpha-amino protecting group is selectively removed to allow subsequent coupling reactions to take place at the amino-terminus. The conditions for the removal of the alpha-amino protecting group do not cause deprotection of the side-chain protecting groups.

The alpha-amino protecting groups are those known in the art of stepwise synthesis of peptides, comprising groups like acyl type protecting groups (e.g. formyl, trifluoroacetyl, acetyl), aromatic urethane type protecting groups (e.g. benzyloxycarbonyl (Cbz) and substituted benzyloxycarbonyl), aliphatic urethane protecting groups (e.g. t-butyloxycarbonyl (Boc), isopropyloxycarbonyl, cyclohexyloxycarbonyl) and alkyl type protecting groups (e.g. benzyl, triphenylmethyl). The preferred protecting group is Boc. The side-chain protecting groups for Tyr include tetrahydropyranyl, tert.-butyl, trityl, benzyl, Cbz, 4-Br-Cbz and 2,6-dichlorobenzyl. The preferred side-chain protecting group for Tyr is 2,6-dichlorobenzyl. The side-chain protecting groups for Asp include benzyl, 2,6-dichlorobenzyl, methyl, ethyl and cyclohexyl. The preferred side-chain protecting group for Asp is cyclohexyl. The side-chain protecting groups for Thr and Ser include acetyl, benzoyl, trityl, tetrahydropyranyl, benzyl, 2,6-dichlorobenzyl and Cbz. The preferred protecting group for Thr and Ser is benzyl. The side-chain protecting groups for Arg include nitro, tosyl (Tos), Cbz, adamantyloxycarbonyl or Boc. The preferred protecting group for Arg is Tos. The side-chain amino group of Lys may be protected with Cbz, 2-Cl-Cbz, Tos or Boc. The 2-Cl-Cbz is the preferred protecting group for Lys. The selection of the side-chain protecting groups is based on the following: The side-chain protecting group remains intact during coupling and is not split off during the deprotection of the amino-terminal protecting group or during coupling conditions. The side-chain protecting group must be removable after completion of the synthesis of the final peptide by reaction conditions that will not alter said peptide.

Solid phase synthesis is usually carried out from the carboxy-terminus of the peptide to be synthesized by coupling the alpha-amino protected (side-chain protected) amino acid to a suitable solid support. An ester linkage is formed when the attachment is made to a chloromethylated or hydroxymethyl resin and the resultant target peptide will have a free carboxyl group at the C-terminus. Alternatively, a benzhydrylamine or p-methylbenzhydrylamine resin is used in which case an amide bond is formed and the resultant target peptide will have a carboxamide group at the C-terminus. These resins are commercially available and their preparation is described by Stewart et al., "Solid Phase Peptide Synthesis" (2nd Edition, Pierce Chemical Co., Rockford, IL., 1984).

In the case of Arg as the C-terminal amino acid (protected at the side-chain with Tos and at the alpha-amino function with Boc) said amino acid can be coupled to the benzhydrylamine resin using various activating agents including dicyclohexylcarbodiimide (DCC), N,N'-diisopropylcarbodiimide or carbonyldiimidazole. Following the attachment to the resin support the alpha-amino protecting group is removed using trifluoroacetic acid (TFA) or HCl in dioxane at a temperature between 0° and 25°. Dimethylsulfide is added to the TFA after the introduction of methionine (Met) to suppress possible S-alkylation. After removal of the alpha-amino protecting group the remaining protected amino acids are coupled stepwise in the required order to obtain the desired peptide sequence. Various activating agents can be used for the coupling reactions including DCC, N,N'-diisopropylcarbodiimide, (benzotriazol-1-yl-oxy)[tris(dimethylamino)]-phosphonium hexafluorophosphate (BOP) and DCC-hydroxybenzotriazole (HOBt). Each protected amino acid is used in excess (>2.5 equivalents) and the couplings are usually carried out in dimethylformamide (DMF), $CH_2Cl_2$ or mixtures thereof. The extent of completion of the coupling reaction is monitored at each stage by the ninhydrin reaction as described by Kaiser et al., Anal. Biochem., <u>34</u>, 595 (1970). In cases of incomplete coupling said coupling reaction is repeated. The coupling reactions can be performed automatically for example on synthesizers like the Vega 250, Applied Biosystems synthesizer or other commercially available instruments. The protocol for a typical synthetic cycle in the synthesis of the peptides of the invention is given on Table 1.

Table 1

| Protocol for a Typical Synthetic Cycle[a] | | | |
|---|---|---|---|
| Step | | Reagent | Time |
| 1 | | 1% DMS/$CH_2Cl_2$ | 1 x 1 min |
| 2 | | 50% TFA/$CH_2Cl_2$ + 1% DMS (v/v) | 1 x 1 min |
| 3 | | 1% DMS/$CH_2Cl_2$ | 1 x 1 min |
| 4 | | 50% TFA/$CH_2Cl_2$ + 1% DMS (v/v) | 1 x 20 min |
| 5 | | $CH_2Cl_2$ | 3 x 1 min[b] |
| 6 | | 10% DIEA/$CH_2Cl_2$ | 1 x 5 min |
| 7 | | $CH_2Cl_2$ | 2 x 1 min |
| 8 | | Repeat Steps 6,7 | |
| 9 | | MeOH | 2 x 1 min |
| 10 | | $CH_2Cl_2$ | 3 x 1 min |
| 11 | a | 2.5 eq. Boc-AA-COOH/$CH_2Cl_2$ | 5 min[c] |
| | b | 2.5 eq. DCC/$CH_2Cl_2$ | 60 min |
| | c | 1.5% DIEA/$CH_2Cl_2$ | 15 min |
| 12 | | Repeat Steps 10,11 | |
| 13 | | $CH_2Cl_2$ | 2 x 1 min |
| 14 | | MeOH | 1 x 1 min |
| 15 | | $CH_2Cl_2$ | 1 x 1 min[b] |

a Solvents for all washings and couplings were measured to volumes of 15-20 ml/g resin
b Kaiser ninhydrin test
c DMF/$CH_2Cl_2$ for Boc-Arg(Tos)-OH

After the entire assemblage of the peptide to be synthesized the peptide-resin is reacted first with TEA/dithioethane and second with a reagent such as liquid HF for 1-2 hours at 0° which cleaves the peptide from the resin and removes all side-chain protecting groups.

Side-chain to side-chain cyclization on the solid support requires the use of an orthogonal protection scheme which enables selective cleavage of the side-chain functions of acidic amino acids (e.g. Asp) and the basic amino acid (e.g. Lys). The 9-fluorenylmethyl (OFm) protecting group for the side-chain of Asp and the 9-fluorenylmethoxycarbonyl (Fmoc) protecting group for the side-chain of Lys can be used for this purpose. In these cases the side-chain protecting groups (OFm and Fmoc) of the Boc-protected peptide-resin are selectively removed with piperidine in DMF. Cyclization is achieved on the solid support using various activating agents including DCC, DCC/HOBt or BOP. The HF reaction is carried out on the cyclized peptide-resin as described above.

Purification of the polypeptides of the invention can be effected using procedures well known in peptide chemistry. The polypeptides of the present invention may be purified using preparative hplc; however, other known chromatographic procedures such as gel permeation, ion exchange and partition chromatography or countercurrent distribution can also be employed.

The instant invention can be used to stimulate the release of growth hormone in a subject by administering to said subject an effective amount of peptides according to Formula I.

The polypeptides of this invention have growth hormone releasing activity. Pharmaceutical compositions in accordance with the invention include analogs of about 29 to 44 amino acids in length, or a nontoxic salt of any of these, dispersed in a pharmaceutically or veterinarily acceptable liquid or solid carrier. Such pharmaceutical compositions can be used for therapeutic or diagnostic purposes in human and veterinary medicine. They are useful for example, in the treatment of growth-related disorders such as pituitary dwarfism and diabetes resulting from abnormal growth hormone production. Furthermore, they can also be used to stimulate the growth or enhance feed efficiency of animals raised for meat production, to enhance milk production and stimulate egg production.

Appropriate dosages of the polypeptides of the invention to be administered will depend on the individual subject and more specifically upon the degree of insufficiency of growth hormone production and the condition of treatment. A person skilled in the art will be able to determine appropriate dosages based on the known circulating levels of growth hormone associated with normal growth and the growth hormone releasing activity of, the polypeptides of the invention. More specific, a dosage range of from 0.04 $\mu$g/kg/day to about 20.0 $\mu$g/kg/day based on body weight of the subject may be used to stimulate release of growth hormone. The dosages employed to stimulate growth activity in livestock will be significantly higher (per kg. of subject weight) than the dosages employed to restore normal growth in cases of growth hormone deficiencies such as pituitary dwarfism in humans. In livestock generally a dosage in the range of from 0.4 $\mu$g/kg/day to about 100$\mu$g/kg/day subcutaneously may be used to stimulate release of pituitary growth hormone.

Thus, the present invention can be used to treat growth-related disorders characterized by insufficient production of growth hormone which comprises administering an amount of the analogs of this invention sufficient to stimulate the production of growth hormone to levels associated with normal growth.

Normal levels of growth hormone vary considerably among individuals and, for any given individual, levels of circulating growth hormone vary considerably during the course of a day. In adult humans, normal serum levels of growth hormone have been reported to vary from about 0-10 nanograms/ml. In children, normal serum levels of growth hormone have been reported to vary from about 0-20 nanograms/ml.

In order to treat hypopituitary dwarfism effectively with the described analogs, treatment is conducted during the period of normal growth. In females, this period generally does not extend far beyond the onset of menses. Thus, treatment of females should be effected approximately from the age of 12 to 16 years, depending upon the individual. In males, the stimulation of growth may be possible for a considerably longer period of time beyond puberty. Thus, effective treatment of males will normally be possible up to about 18 to 19 years of age and, in some individual cases, up to about 25 years.

There is also provided a method of increasing the growth rate of animals by administering an amount of the analog sufficient to stimulate the production of growth hormone at a level greater than that associated with normal growth.

The polypeptides of the invention can be administered in the form of human or veterinary pharmaceutical compositions which can be prepared by conventional pharmaceutical formulation techniques. Compositions suitable for oral, intravenous, subcutaneous, intramuscular, intraperitoneal intranasal, or transdermal administration may be employed. A suitable dosage form for pharmaceutical use is from about 0.01 to about 0.5 mg of the compound of the invention, which may be lyophilized for reconstitution with sterile water or saline. The composition should be maintained at a pH below about 5.0 in order to maintain the stability of the analog. Serum albumin from the species being treated (e.g. human serum albumin in the case of humans, bovine serum albumin in the case of cows and so forth) may also be present together with other known pharmaceutical adjuvants.

The present invention will be described in connection with the following examples which are set forth for the purposes of illustration only.

Example I

Synthesis of Cyclo$^{8,12}$[Asp$^8$,Ala$^{15}$]-GRF(1-29)-NH$_2$

Preparation of Boc-Arg(Tos)-Benzhydrylamine-resin

Benzhydrylamine-resin (60 g, 0.7 meq/g, 42 meq) was washed with 900 ml each of $CH_2Cl_2$, MeOH, $CH_2Cl_2$, 25% Et$_3$N in $CH_2Cl_2$ (3 times), $CH_2Cl_2$, MeOH and $CH_2Cl_2$. Boc-Arg(Tos)-OH (35.95 g, 84 mmol, 2 eq) in DMF (80 ml)-$CH_2Cl_2$ (600 ml) was added, shaken for 5 min and DCC (17.3 g, 84 mmol, 2 eq) added and reacted for 24 hours. The resultant Boc-Arg(Tos)-BHA-resin was washed with DMF, $CH_2Cl_2$, MeOH and $CH_2Cl_2$. Amino acid analysis of an aliquot revealed a substitution of 0.38 mmol/g. The resin was acetylated with 300 ml of 50% Ac$_2$O-pyridine for 2h at 25° and washed with $CH_2Cl_2$, MeOH, $CH_2Cl_2$ and dried in vacuo to give 70 g of Boc-Arg(Tos)-BHA-resin (substitution: 0.6 meq/g).

Preparation of [Asp[8] (OFm),Lys[12](Fmoc),Ala[15] ]-GRF(1-29)-benzhydrylamine-resin

The Boc-Arg(Tos)-BHA-resin (70 g, 0.6 meq/g, 42 meq) was deprotected and neutralized according to the protocol given on Table 1. Trifunctional amino acids were protected as follows: Boc-Arg(Tos), Boc-Asp-(OcHex), Boc-Glu-(OBzl), Boc-Lys(2Cl-Z), Boc-Ser(Bzl), Boc-Thr(Bzl), Boc-Tyr-(2,6-Cl$_2$Bzl), Boc-Asp[8](OFm) and Boc-Lys[12](Fmoc). Exceptions to the protocol were for the coupling of Boc-Gln-OH (positions 24 and 16) and for the coupling immediately following Gln (positions 23 and 15) in which 3 equiv. of the symmetric anhydride was used in DMF (double coupled). In special cases in which a third coupling was required the HOBt ester was prepared using 2.5 equiv. each of Boc-amino acid, l-hydroxybenzotriazole and DCC preactivated in DMF (120 ml), filtered (removal of dicyclohexylurea) and diluted with toluene to 1.21. Coupling was proceeded for 2 hours and 1.5% diisopropylethylamine was added and reacted for additional 15 min..

A portion of the intermediate [Ala[15] ]-GRF(13-29)-BHA-resin (1.88 g, 0.714 mmol) was removed and stepwise solid phase synthesis continued as described above. After incorporation of Lys[12](Fmoc), Step 6 was replaced with 0.6% DIEA/CH$_2$Cl$_2$. The coupling with Boc-Asn-OH (6 equiv.) was carried out by preactivation with l-hydroxybenzotriazole (6.6 equiv.) and DCC (6 equiv.).

Preparation of Cyclo[8,12][Asp[8],Ala[15] ]-GRF(1-29)-NH$_2$

Following the assemblage of Boc-[Asp[8](OFm),Lys[12](Fmoc), Ala[15] ]-GRF(1-29)-BHA-resin (2.72 g, 0.714 mmol) the resin was deprotected with 20% piperidine/DMF for 20 min to give Boc-[Asp[8],Ala[15] ]-GRF(1-29)-BHA-resin. A portion (1.40 g, 0.40 mmol) was cyclized by reaction with BOP reagent (443 mg, 1.0 mmol, 2.5 eq) in DMF (40 ml) containing Et$_3$N (157 $\mu$l, 1.12 mmol, 2.8 eq) for 4 hours. After washing, cyclization was repeated two more times for 11 hours and 3 hours (negative Kaiser ninhydrin test) and the peptide-resin washed, dried and cleaved with HF (~ 20 ml) containing DTE (1 ml/g resin) at 0° for 2 hours. Evaporation of HF was followed by washing with EtOAc, extraction with TFA (6 x 5 ml), evaporation and trituration with ether.

Purification and Characterization of Cyclo[8,12][Asp[8], Ala[15] ]-GRF(1-29)-NH$_2$

The crude product (708 mg) was suspended in 20 ml of H$_2$O (0.025% TFA), stirred, centrifuged, filtered and applied on a Synchropak RP-P column (2.0 x 50 cm) [Elution conditions: eluant (A) H$_2$O (0.025% TFA) - (B) ACN (0.025% TFA); linear gradient 20-45% (B) in 120 min., flow rate 4 ml/min]. Fractions were collected at 1 min intervals. Fractions 82-89 were pooled and lyophilized to give 84 mg of semi-pure product. Side-bands were obtained from fractions 90-93 (33 mg). The semi-pure material (84 mg) was repurified on a Nucleosil C$_{18}$ column (1.0 x 50 cm; 5$\mu$) [Elution conditions: eluant (A) H$_2$O (0.1% TFA) - (B) ACN (0.1% TFA); linear gradient 20-40% (B) in 120 min; flow rate 3 ml/min]. Fractions were collected at 1 min. intervals. Fractions 120-121 were pooled and lyophilized to give a homogeneous product (9 mg) and fractions 122-38 gave 42 mg of a product that was > 97% pure.

The product was shown to be homogeneous by analytical hplc. Amino acid analysis (6M HCl, 110°, 24h): Asp, 2.72; Thr, 0.96; Ser, 2.98; Glu, 2.14; Ala, 4.00; Val, 0.96; Met, 0.97; Ile, 1.89; Leu, 4.28; Tyr, 2.01; Phe, 0.98; Lys, 2.06; Arg, 3.04.

Example 2

Synthesis of Cyclo[8,12][desNH$_2$Tyr[1],D-Ala[2],Asp[8], Ala[15] ]-GRF(1-29)-NH$_2$

A portion of intermediate [Ala[15] ]-GRF(13-29)-BHA- resin (5.1 g, 1.63 mmol) was removed and stepwise solid phase synthesis continued according to the protocol given in Table 1. After incorporation of Lys[12]-(Fmoc), Step 6 was replaced with 0.6% DIEA/CH$_2$Cl$_2$. The synthesis was continued to give 6.4 g of Boc-[Asp[8](OFm),Lys[12](Fmoc),Ala[15] ]- GRF(3-29)-BHA-resin. A 1.0 g (0.255 mmol) portion was subjected to 1 cycle of solid phase synthesis with Boc-D-Ala-OH to give Boc-[D-Ala[2],Asp[8](OFm),Lys[12](Fmoc),Ala[15] ]-GRF(2-29)-BHA-resin. A 0.5 g portion was subjected to a final cycle with desNH$_2$Tyr-OH to give [desNH$_2$Tyr[1],D-Ala[2],Asp[8](OFm),Lys[12](Fmoc),Ala[15] ]-GRF(1-29)-BHA-resin which was deprotected with 20% piperidine/DMF for 20 min and cyclized with BOP reagent (170 mg, 0.384 mmol, 3 eq) in DMF (20 ml) containing DIEA (0.3 ml, 2.2mmol) for 2 hours. The cyclization was repeated with fresh BOP reagent (negative Kaiser ninhydrin test) and the peptide washed, dried and cleaved (as above) with HF (~ 10 ml) at 0° for 2 hours. Evaporation of HF was followed by washing with EtOAc, extraction with TFA, evaporation and trituration with

8

ether to give 229 mg crude product. Purification was carried out by preparative hplc as described above for Cyclo$^{8,12}$[Asp$^8$, Ala$^{15}$]-GRF(1-29)-NH$_2$.

Example 3

Synthesis of Cyclo$^{8,12}$[desNH$_2$ Tyr$^1$,Asp$^8$,Ala$^{15}$]-GRF(1-29)-NH$_2$

A 1.0 g (0.255 mmol) portion of Boc-[Asp$^8$(OFm), Lys$^{12}$-(Fmoc),Ala$^{15}$]-GRF(3-29)-BHA-resin was subjected to 2 cycles of solid phase synthesis with Boc-L-Ala-OH followed by desNH$_2$ Tyr-OH to give [desNH$_2$ Tyr$^1$,Asp$^8$(OFm),Lys$^{12}$(Fmoc),Ala$^{15}$]-GRF(1-29)-BHA-resin which was deprotected with 20% piperidine/DMF for 20 min and cyclized with BOP reagent (170 mg, 0.384 mmol, 3 eq) in DMF (20 ml) containing DIEA (0.3 ml, 2.2mmol) for 2 hours. The cyclization was repeated with fresh BOP reagent (negative Kaiser ninhydrin test) and the peptide washed, dried and cleaved (as above) with HF (~ 10 ml) at 0° for 2 hours. Evaporation of HF was followed by washing with EtOAc, extraction with TFA, evaporation and trituration with ether to give 450 mg crude product. Purification was carried out by preparative hplc as described above for Cyclo$^{8,12}$[Asp$^8$,Ala$^{15}$]-GRF(1-29)-NH$_2$.

Example 4

Synthesis of Cyclo$^{8,12}$[D-Ala$^2$,Asp$^8$,Ala$^{15}$]-GRF(1-29)-NH$_2$

A 0.5 g portion of Boc-[D-Ala$^2$,Asp$^8$(OFm), Lys$^{12}$(Fmoc),Ala$^{15}$]-GRF(2-29)-BHA-resin from Example 2 was subjected to a final cycle with Boc-Tyr[2,6-Cl$_2$ Bzl]-OH to give Boc-[D-Ala$^2$,Asp$^8$(OFm),Lys$^{12}$(Fmoc), Ala$^{15}$]-GRF(1-29)-BHA-resin which was deprotected with 20% piperidine/DMF for 20 min and cyclized with BOP reagent (170 mg, 0.384 mmol, 3 eq) in DMF (20 ml) containing DIEA (0.3 ml, 2.2mmol) for 2 hours. The cyclization was repeated with fresh BOP reagent (negative Kaiser ninhydrin test) and the peptide washed, dried and cleaved (as above) with HF (~ 10 ml) at 0° for 2 hours. Evaporation of HF was followed by washing with EtOAc, extraction with TFA, evaporation and trituration with ether to give 198 mg crude product. Purification was carried out by preparative hplc as described above for Cyclo$^{8,12}$[Asp$^8$,Ala$^{15}$]-GRF-(1-29)-NH$_2$.

Example 5

Synthesis of Cyclo$^{8,12}$[N-Methyl-Tyr$^1$,D-Ala$^2$,Asp$^8$,Ala$^{15}$] -GRF(1-29)-NH$_2$

A 0.5 g portion of Boc-[D-Ala$^2$,Asp$^8$(OFm),-Lys$^{12}$(Fmoc),Ala$^{15}$]-GRF(2-29)BHA-resin (from above) was subjected to a final cycle with Boc-N-Methyl-L-Tyr-(2,6-Cl$_2$ Bzl)-OH to give Boc-[N-Methyl-L-Tyr$^1$(2,6-Cl$_2$ Bzl),D-Ala$^2$,Asp$^8$(OFm),Lys$^{12}$(Fmoc),Ala$^{15}$]-GRF(1-29)-BHA-resin which was deprotected with 20% piperidine/DMF for 20 min and cyclized with BOP reagent (170 mg, 0.384 mmol, 3 eq) in DMF (5 ml) containing DIEA (0.103 ml, 0.765mmol, 6 eq) for 2 hours. The cyclization was repeated 2 more times with fresh BOP reagent (negative ninhydrin test) and the peptide washed, dried and cleaved (as above) with HF (~ 10 ml) at 0° for 2 hours. Evaporation of HF was followed by washing with EtOAc, extraction with TFA, evaporation and trituration with ether to give 219 mg crude product. Purification was carried out by preparative hplc as described above for Cyclo$^{8,12}$[Asp$^8$,Ala$^{15}$]-GRF(1-29)-NH$_2$.

While the invention has been described in connection with the preferred embodiment, it is not intended to limit the scope of the invention to the particular form set forth, but, on the contrary it is intended to cover such alternatives, modifications, and equivalents as may be included within the spirit and scope of the invention as defined by the appended claims.

9

**Claims**

**Claims for the following Contracting States : AT, BE, CH, DE, FR, GB, IT, LI, LU, NL, SE**

1. A cyclic peptide of the formula

```
R1-R2-Asp-Ala-Ile-Phe-Thr-A-Ser-Tyr-Arg-B-Val-Leu-R3-Gln

-Leu-Ser-Ala-Arg-Lys-Leu-Leu-Gln-Asp-Ile-R4-R5-R6-X
```

wherein

| | | |
|---|---|---|
| $R^1$ = | Tyr, desNH$_2$-Tyr, Ac-Tyr, His, N-Methyl-L-Tyr | |
| $R^2$ = | Ala, D-Ala, N-Methyl-D-Ala | |
| $R^3$ = | Gly, Ala, Leu, Val, Ile, Nle, NVal, $\beta$-Ala, $\alpha$-Aib | |
| $R^4$ = | Met, Leu, Nle, Ile | |
| $R^5$ = | Ser, Asn | |
| $R^6$ = | an amino acid sequence selected from the group consisting of Arg-Gln-Gln-Gly-Glu-Ser-Asn-Gln-Glu-Arg-Gly-Ala-Arg-Ala-Arg-Leu or fragments thereof where the fragment is reduced in number by 1 to 15 amino acids from the carboxyl end | |
| X = | OH, NH$_2$, N(R$^7$)(R$^8$) where R$^7$ and R$^8$ = H or lower alkyl | |
| A = | Asp, Glu, $\alpha$-aminopimelic acid, $\alpha$-aminoadipic acid | |
| B = | Lys, Orn, diaminopropionic acid, diaminobutyric acid | |

with the side chain carboxy terminus of A is bonded via an amide bond to the side chain amino terminus of B,

and the pharmaceutically acceptable salts thereof.

2. The peptide of Claim 1 wherein $R^1$ = Tyr, desNH$_2$-Tyr, N-Methyl-L-Tyr; $R^2$ = Ala, D-Ala; $R^3$ = Ala; and X = NH$_2$.

3. The peptide of Claim 2 wherein A = Asp; B = Lys (and the side-chain carboxy terminus of Asp is covalently linked to the side-chain amino terminus of Lys as an amide bond); $R^4$ = Met; $R^5$ = Ser; and $R^6$ = Arg.

4. The peptide of Claim 3 wherein $R^1$ = Tyr.

5. The peptide of Claim 4 wherein $R^2$ = Ala said compound having the formula:

```
Tyr-Ala-Asp-Ala-Ile-Phe-Thr-Asp-Ser-Tyr-Arg-Lys-Val-Leu-Ala-

Gln-Leu-Ser-Ala-Arg-Lys-Leu-Leu-Gln-Asp-Ile-Met-Ser-Arg-NH2.
```

6. The compound of Claim 4 wherein $R^2$ = D-Ala said compound having the formula:

```
Tyr-D-Ala-Asp-Ala-Ile-Phe-Thr-Asp-Ser-Tyr-Arg-Lys-Val-Leu-Ala-

Gln-Leu-Ser-Ala-Arg-Lys-Leu-Leu-Gln-Asp-Ile-Met-Ser-Arg-NH2.
```

7. The compound of Claim 3 wherein $R^1$ = desNH$_2$-Tyr.

**8.** The compound of Claim 7 wherein $R^2$ = Ala said compound having the formula:

```
desNH2-Tyr-Ala-Asp-Ala-Ile-Phe-Thr-Asp-Ser-Tyr-Arg-Lys-Val-
Leu-Ala-Gln-Leu-Ser-Ala-Arg-Lys-Leu-Leu-Gln-Asp-Ile-Met-Ser-
Arg-NH2.
```

**9.** The compound of Claim 7 wherein $R^2$ = D-Ala said compound having the formula:

```
desNH2-Tyr-D-Ala-Asp-Ala-Ile-Phe-Thr-Asp-Ser-Tyr-Arg-Lys-
Val-Leu-Ala-Gln-Leu-Ser-Ala-Arg-Lys-Leu-Leu-Gln-Asp-Ile-Met-
Ser-Arg-NH2.
```

**10.** The compound of Claim 3 wherein $R^1$ = N-Methyl-L-Tyr.

**11.** The compound of claim 10 wherein $R^2$ = Ala said compound having the formula:

```
N-Methyl-L-Tyr-Ala-Asp-Ala-Ile-Phe-Thr-Asp-Ser-Tyr-Arg-Lys-
Val-Leu-Ala-Gln-Leu-Ser-Ala-Arg-Lys-Leu-Leu-Gln-Asp-Ile-Met-
Ser-Arg-NH2
```

**12.** The compound of Claim 10 wherein $R^2$ = D-Ala said compound having the formula:

```
N-Methyl-L-Tyr-D-Ala-Asp-Ala-Ile-Phe-Thr-Asp-Ser-Tyr-Arg-Lys-
Val-Leu-Ala-Gln-Leu-Ser-Ala-Arg-Lys-Leu-Leu-Gln-Asp-Ile-Met-
Ser-Arg-NH2.
```

**13.** Compounds as claimed in any one of claims 1 to 12 for use as therapeutically active substances.

**14.** Use of compounds as claimed in any one of claims 1-12 for the manufacture of a medicament for the treatment of growth-related disorders characterized by growth hormone deficiencies in humans.

**15.** A process for the preparation of compounds as claimed in any one of claims 1-12 characterized in that a duly side-chain protected resin bound polypeptide of corresponding amino acid sequence which has been synthesized by solid phase peptide synthesis is reacted with liquid HF and, if desired, converting such a peptide into a pharmaceutically acceptable salt.

**16.** A pharmaceutical composition containing an effective amount of a compound as claimed in any one of claims 1 to 12 and a non-toxic pharmaceutically acceptable liquid or solid carrier.

**17.** The use of a compound as claimed in any one of claims 1-12 in the treatment of animals in order to promote their growth.

**Claims for the following Contracting State : ES**

1. A process for the preparation of a peptide of the formula

$$R^1-R^2-Asp-Ala-Ile-Phe-Thr-A-Ser-Tyr-Arg-B-Val-Leu-R^3-Gln$$
$$-Leu-Ser-Ala-Arg-Lys-Leu-Leu-Gln-Asp-Ile-R^4-R^5-R^6-X$$

wherein

$R^1$ = Tyr, desNH$_2$-Tyr, Ac-Tyr, His, N-Methyl-L-Tyr
$R^2$ = Ala, D-Ala, N-Methyl-D-Ala
$R^3$ = Gly, Ala, Leu, Val, Ile, Nle, NVal, $\beta$-Ala, $\alpha$-Aib
$R^4$ = Met, Leu, Nle, Ile
$R^5$ = Ser, Asn
$R^6$ = an amino acid sequence selected from the group consisting of Arg-Gln-Gln-Gly-Glu-Ser-Asn-Gln-Glu-Arg-Gly-Ala-Arg-Ala-Arg-Leu or fragments thereof where the fragment is reduced in number by 1 to 15 amino acids from the carboxyl end
$X$ = OH, NH$_2$, N($R^7$)($R^8$) where $R^7$ and $R^8$ = H or lower alkyl
$A$ = Asp, Glu, $\alpha$-aminopimelic acid, $\alpha$-aminoadipic acid
$B$ = Lys, Orn, diaminopropionic acid, diaminobutyric acid

with the side chain carboxy terminus of A is bonded via an amide bond to the side chain amino terminus of B.

and the pharmaceutically acceptable salts thereof characterized in that a duly side-chain protected resin bound polypeptide of corresponding amino acid sequence which has been synthesized by solid phase peptide synthesis is reacted with liquid HF and, if desired, converting such a peptide into a pharmaceutically acceptable salt.

2. A process according to Claim 1 wherein $R^1$ = Tyr, desNH$_2$-Tyr, N-Methyl-L-Tyr; $R^2$ = Ala, D-Ala; $R^3$ = Ala; and X = NH$_2$.

3. A process according to Claim 2 wherein A = Asp; B = Lys (and the side-chain carboxy terminus of Asp is covalently linked to the side-chain amino terminus of Lys as an amide bond); $R^4$ = Met; $R^5$ = Ser; and $R^6$ = Arg.

4. A process according to Claim 3 wherein $R^1$ = Tyr.

5. A process according to Claim 4 wherein $R^2$ = Ala said compound having the formula:

$$Tyr-Ala-Asp-Ala-Ile-Phe-Thr-Asp-Ser-Tyr-Arg-Lys-Val-Leu-Ala-$$
$$Gln-Leu-Ser-Ala-Arg-Lys-Leu-Leu-Gln-Asp-Ile-Met-Ser-Arg-NH_2.$$

6. A process according to Claim 4 wherein $R^2$ = D-Ala said compound having the formula:

$$Tyr-D-Ala-Asp-Ala-Ile-Phe-Thr-Asp-Ser-Tyr-Arg-Lys-Val-Leu-Ala-$$
$$Gln-Leu-Ser-Ala-Arg-Lys-Leu-Leu-Gln-Asp-Ile-Met-Ser-Arg-NH_2.$$

7. A process according to Claim 3 wherein $R^1$ = desNH$_2$-Tyr.

**8.** A process according to Claim 7 wherein $R^2$ = Ala said compound having the formula:

```
desNH2-Tyr-Ala-Asp-Ala-Ile-Phe-Thr-Asp-Ser-Tyr-Arg-Lys-Val-
Leu-Ala-Gln-Leu-Ser-Ala-Arg-Lys-Leu-Leu-Gln-Asp-Ile-Met-Ser-
Arg-NH2.
```

**9.** A process according to Claim 7 wherein $R^2$ = D-Ala said compound having the formula:

```
desNH2-Tyr-D-Ala-Asp-Ala-Ile-Phe-Thr-Asp-Ser-Tyr-Arg-Lys-
Val-Leu-Ala-Gln-Leu-Ser-Ala-Arg-Lys-Leu-Leu-Gln-Asp-Ile-Met-
Ser-Arg-NH2.
```

**10.** A process according to Claim 3 wherein
$R^1$ = N-Methyl-L-Tyr.

**11.** A process according to claim 10 wherein $R^2$ = Ala said compound having the formula:

```
N-Methyl-L-Tyr-Ala-Asp-Ala-Ile-Phe-Thr-Asp-Ser-Tyr-Arg-Lys-
Val-Leu-Ala-Gln-Leu-Ser-Ala-Arg-Lys-Leu-Leu-Gln-Asp-Ile-Met-
Ser-Arg-NH2
```

**12.** A process according to Claim 10 wherein $R^2$ = D-Ala said compound having the formula:

```
N-Methyl-L-Tyr-D-Ala-Asp-Ala-Ile-Phe-Thr-Asp-Ser-Tyr-Arg-Lys-
Val-Leu-Ala-Gln-Leu-Ser-Ala-Arg-Lys-Leu-Leu-Gln-Asp-Ile-Met-
Ser-Arg-NH2.
```

**13.** A cyclic peptide of the formula

$R^1$-$R^2$-Asp-Ala-Ile-Phe-Thr-A-Ser-Tyr-Arg-B-Val-Leu-$R^3$-Gln-Leu-Ser-Ala-Arg-Lys-Leu-Leu-Gln-Asp-Ile-$R^4$-$R^5$-$R^6$-x

wherein
$R^1$ = Tyr, desNH$_2$-Tyr, Ac-Tyr, His, N-Methyl-L-Tyr
$R^2$ = Ala, D-Ala, N-Methyl-D-Ala
$R^3$ = Gly, Ala, Leu, Val, Ile, Nle, NVal, $\beta$-Ala, $\alpha$-Aib
$R^4$ = Met, Leu, Nle, Ile
$R^5$ = Ser, Asn
$R^6$ = an amino acid sequence selected from the group consisting of
Arg-Gln-Gln-Gly-Glu-Ser-Asn-Gln-Glu-Arg-Gly-Ala-Arg-Ala-Arg-Leu   or   fragments   thereof where the fragment is reduced in number by 1 to 15 amino acids from the carboxyl end

X = OH, $NH_2$, $N(R^7)(R^8)$ where $R^7$ and $R^8$ = H or lower alkyl

A = Asp, Glu, $\alpha$-aminopimelic acid, $\alpha$-amino-adipic acid

B = Lys, Orn, diaminopropionic acid, diamino-butyric acid

with the side chain carboxy terminus of A is bonded via an amide bond to the side chain amino terminus of B,

and the pharmaceutically acceptable salts thereof, for use as a therapeutically active substance.

**14.** The use of a cyclic peptide of the formula

$R^1$-$R^2$-Asp-Ala-Ile-Phe-Thr-A-Ser-Tyr-Arg-B-Val-Leu-$R^3$-Gln-Leu-Ser-Ala-Arg-Lys-Leu-Leu-Gln-Asp-Ile-$R^4$-$R^5$-$R^6$-x

wherein

$R^1$ = Tyr, desNH$_2$-Tyr, Ac-Tyr, His, N-Methyl-L-Tyr

$R^2$ = Ala, D-Ala, N-Methyl-D-Ala

$R^3$ = Gly, Ala, Leu, Val, Ile, Nle, NVal, $\beta$-Ala, $\alpha$-Aib

$R^4$ = Met, Leu, Nle, Ile

$R^5$ = Ser, Asn

$R^6$ = an amino acid sequence selected from the group consisting of
Arg-Gln-Gln-Gly-Glu-Ser-Asn-Gln-Glu-Arg-Gly-Ala-Arg-Ala-Arg-Leu or fragments thereof
where the fragment is reduced in number by 1 to 15 amino acids from the carboxyl end

X = OH, $NH_2$, $N(R^7)(R^8)$ where $R^7$ and $R^8$ = H or lower alkyl

A = Asp, Glu, $\alpha$-aminopimelic acid, $\alpha$-amino-adipic acid

B = Lys, Orn, diaminopropionic acid, diamino-butyric acid

with the side chain carboxy terminus of A is bonded via an amide bond to the side chain amino terminus of B,

and the pharmaceutically acceptable salts thereof, for the manufacture of a pharmaceutical composition for the treatment of growth-related disorders characterized by growth hormone deficiencies in humans.

**15.** The use of a cyclic peptide of the formula

$R^1$-$R^2$-Asp-Ala-Ile-Phe-Thr-A-Ser-Tyr-Arg-B-Val-Leu-$R^3$-Gln-Leu-Ser-Ala-Arg-Lys-Leu-Leu-Gln-Asp-Ile-$R^4$-$R^5$-$R^6$-x

wherein

$R^1$ = Tyr, desNH$_2$-Tyr, Ac-Tyr, His, N-Methyl-L-Tyr

$R^2$ = Ala, D-Ala, N-Methyl-D-Ala

$R^3$ = Gly, Ala, Leu, Val, Ile, Nle, NVal, $\beta$-Ala, $\alpha$-Aib

$R^4$ = Met, Leu, Nle, Ile

$R^5$ = Ser, Asn

$R^6$ = an amino acid sequence selected from the group consisting of
Arg-Gln-Gln-Gly-Glu-Ser-Asn-Gln-Glu-Arg-Gly-Ala-Arg-Ala-Arg-Leu or fragments thereof
where the fragment is reduced in number by 1 to 15 amino acids from the carboxyl end

X = OH, $NH_2$, $N(R^7)(R^8)$ where $R^7$ and $R^8$ = H or lower alkyl

A = Asp, Glu, $\alpha$-aminopimelic acid, $\alpha$-amino-adipic acid

B = Lys, Orn, diaminopropionic acid, diamino-butyric acid

with the side chain carboxy terminus of A is bonded via an amide bond to the side chain amino terminus of B,

and the pharmaceutically acceptable salts thereof, in the treatment of animals in order to promote their growth.

**Patentansprüche**

**Patentansprüche für folgende Vertragsstaaten : AT, BE, CH, DE, FR, GB, IT, LI, LU, NL, SE**

1. Ein zyklisches Peptid mit der Formel

$$R^1\text{-}R^2\text{-Asp-Ala-Ile-Phe-Thr-A-Ser-Tyr-Arg-B-Val-Leu-}R^3\text{-Gln}$$

$$\text{-Leu-Ser-Ala-Arg-Lys-Leu-Leu-Gln-Asp-Ile-}R^4\text{-}R^5\text{-}R^6\text{-X}$$

worin

$R^1$ = Tyr, desNH$_2$-Tyr, Ac-Tyr, His, N-Methyl-L-Tyr

$R^2$ = Ala, D-Ala, N-Methyl-D-Ala

$R^3$ = Gly, Ala, Leu, Val, Ile, Nle, NVal, $\beta$-Ala, $\alpha$-Aib

$R^4$ = Met, Leu, Nle, Ile

$R^5$ = Ser, Asn

$R^6$ = eine Aminosäuresequenz ausgewählt aus der Gruppe bestehend aus Arg-Gln-Gln-Gly-Glu-Ser-Asn-Gln-Glu-Arg-Gly-Ala-Arg-Ala-Arg-Leu oder Fragmenten davon, wobei das Fragment vom Carboxylende um eine bis fünfzehn Aminosäuren, verkürzt ist

X = OH, NH$_2$, N($R^7$)($R^8$) mit $R^7$ und $R^8$ = H oder niederes Alkyl

A = Asp, Glu, $\alpha$-Aminopimelinsäure, $\alpha$-Aminoadipinsäure

B = Lys, Orn, Diaminopropionsäure, Diaminobuttersäure

wobei das Seitenkettencarboxylende von A durch eine Amidbindung an das Seitenkettenaminoende von B gebunden ist, und pharmazeutisch annehmbare Salze davon.

2. Das Peptid von Anspruch 1, worin $R^1$ = Tyr, desNH$_2$-Tyr, N-Methyl-L-Tyr; $R^2$ = Ala, D-Ala; $R^3$ = Ala; und X = NH$_2$.

3. Das Peptid von Anspruch 2, worin A = Asp; B = Lys (und das Seitenkettencarboxylende von Asp kovalent als eine Amidbindung an das Seitenkettenaminoende von Lys gebunden ist); $R^4$ = Met; $R^5$ = Ser; und $R^6$ = Arg.

4. Das Peptid von Anspruch 3, worin $R^1$ = Tyr.

5. Das Peptid von Anspruch 4, worin $R^2$ = Ala und diese Verbindung die Formel hat:

$$\text{Tyr-Ala-Asp-Ala-Ile-Phe-Thr-Asp-Ser-Tyr-Arg-Lys-Val-Leu-Ala-}$$

$$\text{Gln-Leu-Ser-Ala-Arg-Lys-Leu-Leu-Gln-Asp-Ile-Met-Ser-Arg-NH}_2.$$

6. Das Peptid von Anspruch 4, worin $R^2$ = D-Ala und diese Verbindung die Formel hat:

$$\text{Tyr-D-Ala-Asp-Ala-Ile-Phe-Thr-Asp-Ser-Tyr-Arg-Lys-Val-Leu-Ala-}$$

$$\text{Gln-Leu-Ser-Ala-Arg-Lys-Leu-Leu-Gln-Asp-Ile-Met-Ser-Arg-NH}_2.$$

7. Die Verbindung von Anspruch 3, worin $R^1$ = desNH$_2$-Tyr.

**8.** Die Verbindung von Anspruch 7, worin $R^2$ = Ala und diese Verbindung die Formel hat:

$$desNH_2\text{-Tyr-Ala-Asp-Ala-Ile-Phe-Thr-Asp-Ser-Tyr-Arg-Lys-Val-}$$

$$\text{Leu-Ala-Gln-Leu-Ser-Ala-Arg-Lys-Leu-Leu-Gln-Asp-Ile-Met-Ser-Arg-}NH_2.$$

**9.** Die Verbindung von Anspruch 7, worin $R^2$ = D-Ala und diese Verbindung die Formel hat:

$$desNH_2\text{-Tyr-D-Ala-Asp-Ala-Ile-Phe-Thr-Asp-Ser-Tyr-Arg-Lys-Val-}$$

$$\text{Leu-Ala-Gln-Leu-Ser-Ala-Arg-Lys-Leu-Leu-Gln-Asp-Ile-Met-Ser-Arg-}NH_2.$$

**10.** Die Verbindung von Anspruch 3, worin $R^1$ = N-Methyl-L-Tyr.

**11.** Die Verbindung von Anspruch 10, worin $R^2$ = Ala und diese Verbindung die Formel hat:

$$\text{N-Methyl-L-Tyr-Ala-Asp-Ala-Ile-Phe-Thr-Asp-Ser-Tyr-Arg-Lys-Val-}$$

$$\text{Leu-Ala-Gln-Leu-Ser-Ala-Arg-Lys-Leu-Leu-Gln-Asp-Ile-Met-Ser-Arg-}NH_2.$$

**12.** Die Verbindung von Anspruch 10, worin $R^2$ = D-Ala und diese Verbindung die Formel hat:

$$\text{N-Methyl-L-Tyr-D-Ala-Asp-Ala-Ile-Phe-Thr-Asp-Ser-Tyr-Arg-Lys-Val-}$$

$$\text{Leu-Ala-Gln-Leu-Ser-Ala-Arg-Lys-Leu-Leu-Gln-Asp-Ile-Met-Ser-Arg-}NH_2.$$

**13.** Verbindungen wie in irgendeinem der Ansprüche 1 bis 12 beansprucht zur Verwendung als therapeutisch aktive Substanzen.

**14.** Verwendung von in irgendeinem der Ansprüche 1 bis 12 beanspruchten Verbindungen zur Herstellung eines Medikaments zur Behandlung von mit dem Wachstum in Verbindung stehenden durch Wachstumshormonmangel bei Menschen charakterisierten Krankheiten.

**15.** Ein Verfahren zur Herstellung von wie in irgendeinem der Ansprüche 1 bis 12 beanspruchten Verbindungen dadurch gekennzeichnet, dass ein ausreichend seitenkettengeschütztes, trägergebundenes Polypeptid mit entsprechender Aminosäuresequenz das durch Festphasenpeptidsynthese synthetisiert worden ist mit flüssigem HF behandelt wird und, falls gewünscht, dass ein solches Peptid in ein pharmazeutisch sinnvolles Salz überführt wird.

**16.** Eine pharmazeutische Zusammensetzung, die eine wirksame Menge einer wie in irgendeinem der Ansprüche 1 bis 12 beanspruchten Verbindung und einen nicht toxischen pharmazeutisch annehmbaren flüssigen oder festen Träger enthält.

**17.** Die Verwendung einer wie in irgendeinem der Ansprüche 1 bis 12 beanspruchten Verbindung zur Behandlung von Tieren, um ihr Wachstum zu fördern.

**Patentansprüche für folgenden Vertragsstaat : ES**

1. Ein Verfahren zur Herstellung eines Peptids mit der Formel

$$R^1\text{-}R^2\text{-Asp-Ala-Ile-Phe-Thr-A-Ser-Tyr-Arg-B-Val-Leu-}R^3\text{-Gln}$$

$$\text{-Leu-Ser-Ala-Arg-Lys-Leu-Leu-Gln-Asp-Ile-}R^4\text{-}R^5\text{-}R^6\text{-X}$$

worin

$R^1$ = Tyr, desNH$_2$-Tyr, Ac-Tyr, His, N-Methyl-L-Tyr

$R^2$ = Ala, D-Ala, N-Methyl-D-Ala

$R^3$ = Gly, Ala, Leu, Val, Ile, Nle, NVal, $\beta$-Ala, $\alpha$-Aib

$R^4$ = Met, Leu, Nle, Ile

$R^5$ = Ser, Asn

$R^6$ = eine Aminosäuresequenz ausgewählt aus der Gruppe bestehend aus Arg-Gln-Gln-Gly-Glu-Ser-Asn-Gln-Glu-Arg-Gly-Ala-Arg-Ala-Arg-Leu oder Fragmenten davon, wobei das Fragment vom Carboxylende um eine bis fünfzehn Aminosäuren, verkürzt ist

X = OH, NH$_2$, N(R$^7$)(R$^8$) mit R$^7$ und R$^8$ = H oder niederes Alkyl

A = Asp, Glu, $\alpha$-Aminopimelinsäure, $\alpha$-Aminoadipinsäure

B = Lys, Orn, Diaminopropionsäure, Diaminobuttersäure

wobei das Seitenkettencarboxylende von A durch eine Amidbindung an das Seitenkettenaminoende von B gebunden ist, und pharmazeutisch annehmbare Salze davon, dadurch gekennzeichnet, dass ein ausreichend seitenkettengeschütztes, trägergebundenes Polypeptid mit entsprechender Aminosäuresequenz das durch Festphasenpeptidsynthese synthetisiert worden ist mit flüssigem HF behandelt wird und, falls gewünscht, dass ein solches Peptid in ein pharmazeutisch sinnvolles Salz überführt wird.

2. Ein Verfahren nach Anspruch 1, worin $R^1$ = Tyr, desNH$_2$-Tyr, N-Methyl-L-Tyr; $R^2$ = Ala, D-Ala; $R^3$ = Ala; und X = NH$_2$.

3. Ein Verfahren nach Anspruch 2, worin A = Asp; B = Lys (und das Seitenkettencarboxylende von Asp kovalent als eine Amidbindung an das Seitenkettenaminoende von Lys gebunden ist); $R^4$ = Met; $R^5$ = Ser; und $R^6$ = Arg.

4. Ein Verfahren nach Anspruch 3, worin $R^1$ = Tyr.

5. Ein Verfahren nach Anspruch 4, worin $R^2$ = Ala und besagte Verbindung die Formel hat:

$$\text{Tyr-Ala-Asp-Ala-Ile-Phe-Thr-Asp-Ser-Tyr-Arg-Lys-Val-Leu-Ala-}$$

$$\text{Gln-Leu-Ser-Ala-Arg-Lys-Leu-Leu-Gln-Asp-Ile-Met-Ser-Arg-NH}_2.$$

6. Ein Verfahren nach Anspruch 4, worin $R^2$ = D-Ala und besagte Verbindung die Formel hat:

$$\text{Tyr-D-Ala-Asp-Ala-Ile-Phe-Thr-Asp-Ser-Tyr-Arg-Lys-Val-Leu-Ala-}$$

$$\text{Gln-Leu-Ser-Ala-Arg-Lys-Leu-Leu-Gln-Asp-Ile-Met-Ser-Arg-NH}_2.$$

7. Ein Verfahren nach Anspruch 3, worin $R^1$ = desNH$_2$-Tyr.

8. Ein Verfahren nach Anspruch 7, worin $R^2$ = Ala und besagte Verbindung die Formel hat:

desNH$_2$-Tyr-Ala-Asp-Ala-Ile-Phe-Thr-Asp-Ser-Tyr-Arg-Lys-Val-

Leu-Ala-Gln-Leu-Ser-Ala-Arg-Lys-Leu-Leu-Gln-Asp-Ile-Met-Ser-Arg-NH$_2$.


9. Ein Verfahren nach Anspruch 7, worin $R^2$ = D-Ala und besagte Verbindung die Formel hat:

desNH$_2$-Tyr-D-Ala-Asp-Ala-Ile-Phe-Thr-Asp-Ser-Tyr-Arg-Lys-Val-

Leu-Ala-Gln-Leu-Ser-Ala-Arg-Lys-Leu-Leu-Gln-Asp-Ile-Met-Ser-Arg-NH$_2$.


10. Ein Verfahren nach Anspruch 3, worin $R^1$ = N-Methyl-L-Tyr.

11. Ein Verfahren nach Anspruch 10, worin $R^2$ = Ala und besagte Verbindung die Formel hat:

N-Methyl-L-Tyr-Ala-Asp-Ala-Ile-Phe-Thr-Asp-Ser-Tyr-Arg-Lys-Val-

Leu-Ala-Gln-Leu-Ser-Ala-Arg-Lys-Leu-Leu-Gln-Asp-Ile-Met-Ser-Arg-NH$_2$.


12. Ein Verfahren nach Anspruch 10, worin $R^2$ = D-Ala und besagte Verbindung die Formel hat:

N-Methyl-L-Tyr-D-Ala-Asp-Ala-Ile-Phe-Thr-Asp-Ser-Tyr-Arg-Lys-Val-

Leu-Ala-Gln-Leu-Ser-Ala-Arg-Lys-Leu-Leu-Gln-Asp-Ile-Met-Ser-Arg-NH$_2$.


13. Ein zyklisches Peptid mit der Formel

$R^1$-$R^2$-Asp-Ala-Ile-Phe-Thr-A-Ser-Tyr-Arg-B-Val-Leu-$R^3$-Gln

-Leu-Ser-Ala-Arg-Lys-Leu-Leu-Gln-Asp-Ile-$R^4$-$R^5$-$R^6$-X


worin
$R^1$ = Tyr, desNH$_2$-Tyr, Ac-Tyr, His, N-Methyl-L-Tyr
$R^2$ = Ala, D-Ala, N-Methyl-D-Ala
$R^3$ = Gly, Ala, Leu, Val, Ile, Nle, NVal, $\beta$-Ala, $\alpha$-Aib
$R^4$ = Met, Leu, Nle, Ile
$R^5$ = Ser, Asn
$R^6$ = eine Aminosäuresequenz ausgewählt aus der Gruppe bestehend aus
Arg-Gln-Gln-Gly-Glu-Ser-Asn-Gln-Glu-Arg-Gly-Ala-Arg-Ala-Arg-Leu oder Fragmenten davon, wobei das Fragment vom Carboxylende um eine bis fünfzehn Aminosäuren, verkürzt ist
X = OH, NH$_2$, N($R^7$)($R^8$) mit $R^7$ und $R^8$ = H oder niederes Alkyl

A = Asp, Glu, $\alpha$-Aminopimelinsäure, $\alpha$-Aminoadipinsäure
B = Lys, Orn, Diaminopropionsäure, Diaminobuttersäure
wobei das Seitenkettencarboxylende von A durch eine Amidbindung an das Seitenkettenaminoende von B gebunden ist, und pharmazeutisch annehmbare Salze davon zur Verwendung als therapeutisch wirksame Substanz.

**14.** Die Verwendung eines zyklischen Peptids mit der Formel

$$R^1\text{-}R^2\text{-Asp-Ala-Ile-Phe-Thr-A-Ser-Tyr-Arg-B-Val-Leu-}R^3\text{-Gln}$$

$$\text{-Leu-Ser-Ala-Arg-Lys-Leu-Leu-Gln-Asp-Ile-}R^4\text{-}R^5\text{-}R^6\text{-X}$$

worin
$R^1$ = Tyr, desNH$_2$-Tyr, Ac-Tyr, His, N-Methyl-L-Tyr
$R^2$ = Ala, D-Ala, N-Methyl-D-Ala
$R^3$ = Gly, Ala, Leu, Val, Ile, Nle, NVal, $\beta$-Ala, $\alpha$-Aib
$R^4$ = Met, Leu, Nle, Ile
$R^5$ = Ser, Asn
$R^6$ = eine Aminosäuresequenz ausgewählt aus der Gruppe bestehend aus
Arg-Gln-Gln-Gly-Glu-Ser-Asn-Gln-Glu-Arg-Gly-Ala-Arg-Ala-Arg-Leu oder Fragmenten davon, wobei das Fragment vom Carboxylende um eine bis fünfzehn Aminosäuren, verkürzt ist
X = OH, NH$_2$, N($R^7$)($R^8$) mit $R^7$ und $R^8$ = H oder niederes Alkyl
A = Asp, Glu, $\alpha$-Aminopimelinsäure, $\alpha$-Aminoadipinsäure
B = Lys, Orn, Diaminopropionsäure, Diaminobuttersäure
wobei das Seitenkettencarboxylende von A durch eine Amidbindung an das Seitenkettenaminoende von B gebunden ist, und pharmazeutisch annehmbare Salze davon zur Herstellung einer pharmazeutischen Zusammensetzung zur Behandlung von mit dem Wachstum in Verbindung stehenden durch Wachstumshormonmangel bei Menschen charakterisierten Krankheiten.

**15.** Die Verwendung eines zyklischen Peptids mit der Formel

$$R^1\text{-}R^2\text{-Asp-Ala-Ile-Phe-Thr-A-Ser-Tyr-Arg-B-Val-Leu-}R^3\text{-Gln}$$

$$\text{-Leu-Ser-Ala-Arg-Lys-Leu-Leu-Gln-Asp-Ile-}R^4\text{-}R^5\text{-}R^6\text{-X}$$

worin
$R^1$ = Tyr, desNH$_2$-Tyr, Ac-Tyr, His, N-Methyl-L-Tyr
$R^2$ = Ala, D-Ala, N-Methyl-D-Ala
$R^3$ = Gly, Ala, Leu, Val, Ile, Nle, NVal, $\beta$-Ala, $\alpha$-Aib
$R^4$ = Met, Leu, Nle, Ile
$R^5$ = Ser, Asn
$R^6$ = eine Aminosäuresequenz ausgewählt aus der Gruppe bestehend aus
Arg-Gln-Gln-Gly-Glu-Ser-Asn-Gln-Glu-Arg-Gly-Ala-Arg-Ala-Arg-Leu oder Fragmenten davon, wobei das Fragment vom Carboxylende um eine bis fünfzehn Aminosäuren, verkürzt ist
X = OH, NH$_2$, N($R^7$)($R^8$) mit $R^7$ und $R^8$ = H oder niederes Alkyl
A = Asp, Glu, $\alpha$-Aminopimelinsäure, $\alpha$-Aminoadipinsäure
B = Lys, Orn, Diaminopropionsäure, Diaminobuttersäure
wobei das Seitenkettencarboxylende von A durch eine Amidbindung an das Seitenkettenaminoende von B gebunden ist, und pharmazeutisch annehmbare Salze davon zur Behandlung von Tieren, um ihr Wachstum zu fördern.

EP 0 307 860 B1

**Revendications**
**Revendications pour les Etats contractants suivants : AT, BE, CH, DE, FR, GB, IT, LI, LU, NL, SE**

**1.** Peptide cyclique de formule

$$R^1\text{-}R^2\text{-Asp-Ala-Ile-Phe-Thr-}\overline{A}\text{-Ser-Tyr-Arg-}\overline{B}\text{-Val-Leu-}R^3\text{-Gln-Leu-}$$
$$\text{Ser-Ala-Arg-Lys-Leu-Leu-Gln-Asp-Ile-}R^4\text{-}R^5\text{-}R^6\text{-X}$$

dans laquelle
$R^1$ = Tyr, desNH$_2$-Tyr, Ac-Tyr, His, N-méthyl-L-Tyr
$R^2$ = Ala, D-Ala, N-méthyl-D-Ala
$R^3$ = Gly, Ala, Leu, Val, Ile, Nle, NVal, $\beta$-Ala, $\alpha$-Aib
$R^4$ = Met, Leu, Nle, Ile
$R^5$ = Ser, Asn
$R^6$ = séquence d'acides aminés choisie dans le groupe comprenant
Arg-Gln-Gln-Gly-Glu-Ser-Asn-Gln-Glu-Arg-Gly-Ala-Arg-Ala-Arg-Leu ou des fragments de celle-ci dans lesquels le nombre d'acides aminés du fragment est réduit de 1 à 15 à partir de l'extrémité carboxylique.
X = OH, NH$_2$, N(R$^7$)(R$^8$) dans lequel R$^7$ et R$^8$ = H ou un alkyle inférieur
A = Asp, Glu, acide $\alpha$-aminopimélique, acide $\alpha$-aminoadipique
B = Lys, Orn, acide diaminopropionique, acide diaminobutyrique
avec le carboxy terminal de la chaîne latérale de A qui est lié par l'intermédiaire d'une liaison amide à l'amine terminal de la chaîne latérale de B,
et les sels acceptables du point de vue pharmaceutique de celui-ci.

**2.** Peptide de la revendication 1 dans lequel $R^1$ = Tyr, desNH$_2$-Tyr, N-méthyl-L-Tyr ; $R^2$ = Ala, D-Ala ; $R^3$ = Ala ; et X = NH$_2$.

**3.** Peptide de la revendication 2 dans lequel A = Asp ; B = Lys (et le carboxy terminal de la chaîne latérale d'Asp est lié par covalence à l'amine terminal de la chaîne latérale de Lys sous forme de liaison amide) ;
$R^4$ = Met : $R^5$ = Ser ; et $R^6$ = Arg.

**4.** Peptide de la revendication 3 dans lequel $R^1$ = Tyr.

**5.** Peptide de la revendication 4 dans lequel $R^2$ = Ala, ledit composé ayant pour formule :

$$\text{Tyr-Ala-Asp-Ala-Ile-Phe-Thr-}\overline{\text{Asp-Ser-Tyr-Arg-Lys}}\text{-Val-Leu-Ala-}$$
$$\text{Gln-Leu-Ser-Ala-Arg-Lys-Leu-Leu-Gln-Asp-Ile-Met-Ser-Arg-NH}_2.$$

**6.** Composé de la revendication 4 dans lequel $R^2$ = D-Ala, ledit composé ayant pour formule :

$$\text{Tyr-D-Ala-Asp-Ala-Ile-Phe-Thr-}\overline{\text{Asp-Ser-Tyr-Arg-Lys}}\text{-Val-Leu-Ala-}$$
$$\text{Gln-Leu-Ser-Ala-Arg-Lys-Leu-Leu-Gln-Asp-Ile-Met-Ser-Arg-NH}_2.$$

**7.** Composé de la revendication 3 dans lequel $R^1$ = desNH$_2$-Tyr.

20

**8.** Composé de la revendication 7 dans lequel R$^2$ = Ala, ledit composé ayant pour formule :

desNH$_2$-Tyr-Ala-Asp-Ala-Ile-Phe-Thr-Asp-Ser-Tyr-Arg-Lys-Val-Leu-Ala-Gln-Leu-Ser-Ala-Arg-Lys-Leu-Leu-Gln-Asp-Ile-Met-Ser-Arg-NH$_2$.

**9.** Composé de la revendication 7 dans lequel R$^2$ = D-Ala, ledit composé ayant pour formule :

desNH$_2$-Tyr-D-Ala-Asp-Ala-Ile-Phe-Thr-Asp-Ser-Tyr-Arg-Lys-Val-Leu-Ala-Gln-Leu-Ser-Ala-Arg-Lys-Leu-Leu-Gln-Asp-Ile-Met-Ser-Arg-NH$_2$.

**10.** Composé de la revendication 3 dans lequel R$^1$ = N-méthyl-L-Tyr.

**11.** Composé de la revendication 10 dans lequel R$^2$ = Ala, ledit composé ayant pour formule :

N-méthyl-L-Tyr-Ala-Asp-Ala-Ile-Phe-Thr-Asp-Ser-Tyr-Arg-Lys-Val-Leu-Ala-Gln-Leu-Ser-Ala-Arg-Lys-Leu-Leu-Gln-Asp-Ile-Met-Ser-Arg-NH$_2$.

**12.** Composé de la revendication 10 dans lequel R$^2$ = D-Ala, ledit composé ayant pour formule :

N-méthyl-L-Tyr-D-Ala-Asp-Ala-Ile-Phe-Thr-Asp-Ser-Tyr-Arg-Lys-Val-Leu-Ala-Gln-Leu-Ser-Ala-Arg-Lys-Leu-Leu-Gln-Asp-Ile-Met-Ser-Arg-NH$_2$.

**13.** Composé comme revendiqué dans l'une des revendications 1 à 12 pour utilisation comme substances actives du point de vue thérapeutique.

**14.** Utilisation de composés comme revendiqué dans l'une quelconque des revendications 1-12 pour la fabrication d'un médicament pour le traitement de troubles relatifs à la croissance caractérisés par des déficiences de l'hormone de croissance chez l'homme.

**15.** Procédé pour la préparation de composés comme revendiqué dans l'une quelconque des revendications 1-12, caractérisé en ce qu'un polypeptide aux chaînes latérales dûment protégées, fixé à une résine, de séquence d'acides aminés correspondante, qui a été synthétisé par synthèse peptidique en phase solide, est mis à réagir avec du HF liquide, et, si souhaité, conversion d'un tel peptide en sel acceptable du point de vue pharmaceutique.

**16.** Composition pharmaceutique contenant une quantité efficace d'un composé tel que revendiqué dans l'une quelconque des revendications 1 à 12 et un véhicule liquide ou solide non toxique acceptable du point de vue pharmaceutique.

**17.** Utilisation d'un composé tel que revendiqué dans l'une quelconque des revendications 1-12 dans le traitement d'animaux dans le but de favoriser leur croissance.

**Revendications pour l'Etat contractant suivant : ES**

**1.** Procédé pour la préparation d'un peptide de formule

$$R^1\text{-}R^2\text{-Asp-Ala-Ile-Phe-Thr-A-Ser-Tyr-Arg-B-Val-Leu-}R^3\text{-Gln-Leu-Ser-Ala-Arg-Lys-Leu-Leu-Gln-Asp-Ile-}R^4\text{-}R^5\text{-}R^6\text{-X}$$

dans laquelle

$R^1$ = Tyr, desNH$_2$-Tyr, Ac-Tyr, His, N-méthyl-L-Tyr

$R^2$ = Ala, D-Ala, N-méthyl-D-Ala

$R^3$ = Gly, Ala, Leu, Val, Ile, Nle, NVal, $\beta$-Ala, $\alpha$-Aib

$R^4$ = Met, Leu, Nle, Ile

$R^5$ = Ser, Asn

$R^6$ = séquence d'acides aminés choisie dans le groupe comprenant Arg-Gln-Gln-Gly-Glu-Ser-Asn-Gln-Glu-Arg-Gly-Ala-Arg-Ala-Arg-Leu ou des fragments de celle-ci dans lesquels le nombre d'acides aminés du fragment est réduit de 1 à 15 à partir de l'extrémité carboxylique.

X = OH, NH$_2$, N(R$^7$)(R$^8$) dans lequel R$^7$ et R$^8$ = H ou un alkyle inférieur

A = Asp, Glu, acide $\alpha$-aminopimélique, acide $\alpha$-aminoadipique

B = Lys, Orn, acide diaminopropionique, acide diaminobutyrique

avec le carboxy terminal de la chaîne latérale de A qui est lié par l'intermédiaire d'une liaison amide à l'amine terminal de la chaîne latérale de B,

et les sels acceptables du point de vue pharmaceutique de celle-ci caractérisés en ce qu'un polypeptide aux chaînes latérales dûment protégées, fixé à une résine, de séquence d'acides aminés correspondante, qui a été synthétisé par synthèse peptidique en phase solide, est mis à réagir avec du HF liquide, et, si souhaité, conversion d'un tel peptide en sel acceptable du point de vue pharmaceutique.

**2.** Procédé selon la revendication 1 dans lequel $R^1$ = Tyr, desNH$_2$-Tyr N-méthyl-L-Tyr ; $R^2$ = Ala, D-Ala : $R^3$ = Ala ; et X = NH$_2$.

**3.** Procédé selon la revendication 2 dans lequel A = Asp ; B = Lys ( et le carboxy terminal de la chaîne latérale d'Asp est lié par covalence à l'amine terminal de la chaîne latérale de la lysine sous forme de liaison amide) : $R^4$ = Met ; $R^5$ = Ser ; et $R^6$ = Arg.

**4.** Procédé selon la revendication 3 dans lequel $R^1$ = Tyr.

**5.** Procédé selon la revendication 4 dans lequel $R^2$ = Ala, ledit composé ayant pour formule :

$$\text{Tyr-Ala-Asp-Ala-Ile-Phe-Thr-Asp-Ser-Tyr-Arg-Lys-Val-Leu-Ala-Gln-Leu-Ser-Ala-Arg-Lys-Leu-Leu-Gln-Asp-Ile-Met-Ser-Arg-NH}_2.$$

**6.** Procédé selon la revendication 4 dans lequel $R^2$ = D-Ala, ledit composé ayant pour formule :

Tyr-D-Ala-Asp-Ala-Ile-Phe-Thr-Asp-Ser-Tyr-Arg-Lys-Val-Leu-Ala-Gln-Leu-Ser-Ala-Arg-Lys-Leu-Leu-Gln-Asp-Ile-Met-Ser-Arg-NH$_2$.

**7.** Procédé selon la revendication 3 dans lequel $R^1$ = desNH$_2$-Tyr.

**8.** Procédé selon la revendication 7 dans lequel $R^2$ = Ala, ledit composé ayant pour formule :

desNH$_2$-Tyr-Ala-Asp-Ala-Ile-Phe-Thr-Asp-Ser-Tyr-Arg-Lys-Val-Leu-Ala-Gln-Leu-Ser-Ala-Arg-Lys-Leu-Leu-Gln-Asp-Ile-Met-Ser-Arg-NH$_2$.

**9.** Procédé selon la revendication 7 dans lequel $R^2$ = D-Ala, ledit composé ayant pour formule :

desNH$_2$-Tyr-D-Ala-Asp-Ala-Ile-Phe-Thr-Asp-Ser-Tyr-Arg-Lys-Val-Leu-Ala-Gln-Leu-Ser-Ala-Arg-Lys-Leu-Leu-Gln-Asp-Ile-Met-Ser-Arg-NH$_2$.

**10.** Procédé selon la revendication 3 dans lequel $R^1$ = N-méthyl-L-Tyr.

**11.** Procédé selon la revendication 10 dans lequel $R^2$ = Ala, ledit composé ayant pour formule :

N-méthyl-L-Tyr-Ala-Asp-Ala-Ile-Phe-Thr-Asp-Ser-Tyr-Arg-Lys-Val-Leu-Ala-Gln-Leu-Ser-Ala-Arg-Lys-Leu-Leu-Gln-Asp-Ile-Met-Ser-Arg-NH$_2$.

**12.** Procédé selon la revendication 10 dans lequel $R^2$ = D-Ala, ledit composé ayant pour formule :

N-méthyl-L-Tyr-D-Ala-Asp-Ala-Ile-Phe-Thr-Asp-Ser-Tyr-Arg-Lys-Val-Leu-Ala-Gln-Leu-Ser-Ala-Arg-Lys-Leu-Leu-Gln-Asp-Ile-Met-Ser-Arg-NH$_2$.

**13.** Peptide cyclique de formule

$$R^1\text{-}R^2\text{-Asp-Ala-Ile-Phe-Thr-A-Ser-Tyr-Arg-B-Val-Leu-}R^3\text{-Gln-Leu-}$$
$$\text{Ser-Ala-Arg-Lys-Leu-Leu-Gln-Asp-Ile-}R^4\text{-}R^5\text{-}R^6\text{-X}$$

dans laquelle

$R^1$ = Tyr, desNH$_2$-Tyr Ac-Tyr, His, N-méthyl-L-Tyr

$R^2$ = Ala, D-Ala, N-méthyl-D-Ala

$R^3$ = Gly, Ala, Leu, Val, Ile, Nle, NVal, $\beta$-Ala, $\alpha$-Aib

$R^4$ = Met, Leu, Nle, Ile

$R^5$ = Ser, Asn

$R^6$ = séquence d'acides aminés choisie dans le groupe comprenant Arg-Gln-Gln-Gly-Glu-Ser-Asn-Gln-Glu-Arg-Gly-Ala-Arg-Ala-Arg-Leu ou des fragments de celle-ci dans lesquels le nombre d'acides aminés du fragment est réduit de 1 à 15 à partir de l'extrémité carboxylique.

X = OH, NH$_2$, N(R$^7$)(R$^8$) dans lequel R$^7$ et R$^8$ = H ou un alkyle inférieur

A = Asp, Glu, acide $\alpha$-aminopimélique. acide $\alpha$-aminoadipique

B = Lys, Orn, acide diaminopropionique. acide diaminobutyrique

avec le carboxy terminal de la chaîne latérale de A qui est lié par l'intermédiaire d'une liaison amide à l'amine terminal de la chaîne latérale de B.

et les sels acceptables du point de vue pharmaceutique de celui-ci, pour utilisation comme substances actives du point de vue thérapeutique.

**14.** Utilisation d'un peptide cyclique de formule

$$R^1\text{-}R^2\text{-Asp-Ala-Ile-Phe-Thr-A-Ser-Tyr-Arg-B-Val-Leu-}R^3\text{-Gln-Leu-}$$
$$\text{Ser-Ala-Arg-Lys-Leu-Leu-Gln-Asp-Ile-}R^4\text{-}R^5\text{-}R^6\text{-X}$$

dans laquelle

$R^1$ = Tyr, desNH$_2$-Tyr Ac-Tyr. His, N-méthyl-L-Tyr

$R^2$ = Ala, D-Ala, N-méthyl-D-Ala

$R^3$ = Gly, Ala, Leu, Val, Ile, Nle, NVal, $\beta$-Ala, $\alpha$-Aib

$R^4$ = Met, Leu, Nle, Ile

$R^5$ = Ser, Asn

$R^6$ = séquence d'acides aminés choisie dans le groupe comprenant Arg-Gln-Gln-Gly-Glu-Ser-Asn-Gln-Glu-Arg-Gly-Ala-Arg-Ala-Arg-Leu ou des fragments de celle-ci dans lequel le nombre d'acides aminés du fragment est réduit de 1 à 15 à partir de l'extrémité carboxylique.

X = OH, NH$_2$, N(R$^7$)(R$^8$) dans lequel R$^7$ et R$^8$ = H ou un alkyle inférieur

A = Asp, Glu, acide $\alpha$-aminopimélique, acide $\alpha$-aminoadipique

B = Lys, Orn, acide diaminopropionique, acide diaminobutyrique

avec le carboxy terminal de la chaîne latérale de A qui est lié par l'intermédiaire d'une liaison amide à l'amine terminal de la chaîne latérale de B,

et les sels acceptables du point de vue pharmaceutique de celui-ci, pour la fabrication d'un médicament pour le traitement de troubles relatifs à la croissance caractérisés par les déficiences de l'hormone de croissance chez l'homme.

**15.** Utilisation d'un peptide cyclique de formule

$$R^1\text{-}R^2\text{-}Asp\text{-}Ala\text{-}Ile\text{-}Phe\text{-}Thr\text{-}\overline{A\text{-}Ser\text{-}Tyr\text{-}Arg\text{-}B}\text{-}Val\text{-}Leu\text{-}R^3\text{-}Gln\text{-}Leu\text{-}$$
$$Ser\text{-}Ala\text{-}Arg\text{-}Lys\text{-}Leu\text{-}Leu\text{-}Gln\text{-}Asp\text{-}Ile\text{-}R^4\text{-}R^5\text{-}R^6\text{-}X$$

dans laquelle

$R^1$ = Tyr, desNH$_2$-Tyr, Ac-Tyr, His, H-méthyl-L-Tyr

$R^2$ = Ala, D-Ala, N-méthyl-D-Ala

$R^3$ = Gly, Ala, Leu, Val, Ile, Nle, NVal, $\beta$-Ala, $\alpha$-Aib

$R^4$ = Met, Leu, Nle, Ile

$R^5$ = Ser, Asn

$R^6$ = séquence d'acides aminés choisie dans le groupe comprenant Arg-Gln-Gln-Gly-Glu-Ser-Asn-Gln-Glu-Arg-Gly-Ala-Arg-Ala-Arg-Leu ou des fragments de celle-ci dans lesquels le nombre d'acides aminés du fragment est réduit de 1 à 15 à partir de l'extrémité carboxylique.

X = OH, NH$_2$, N(R$^7$)(R$^8$) dans lequel R$^7$ et R$^8$ = H ou un alkyle inférieur

A = Asp, Glu, acide $\alpha$-aminopimélique, acide $\alpha$-aminoadipique

B = Lys, Orn, acide diaminopropionique, acide diaminobutyrique

avec le carboxy terminal de la chaîne latérale de A qui est lié par l'intermédiaire d'une liaison amide à l'amine terminal de la chaîne latérale de B,

et les sels acceptables du point de vue pharmaceutique de celui-ci. dans le traitement d'animaux dans le but de favoriser leur croissance.

Figure 1

PERIFUSION OF RAT PITUITARY CELLS

2 plts/filter (1:4 dilutions) Perl#16